(19) Europäisches Patentamt / European Patent Office / Office européen des brevets

(11) **EP 4 606 424 A1**

(12) **EUROPÄISCHE PATENTANMELDUNG**

(43) Veröffentlichungstag:
27.08.2025  Patentblatt 2025/35

(21) Anmeldenummer: 25153309.7

(22) Anmeldetag: 22.01.2025

(51) Internationale Patentklassifikation (IPC):
**A61N 5/06** (2006.01)

(52) Gemeinsame Patentklassifikation (CPC):
**A61N 5/0624;** A61N 2005/063; A61N 2005/0643;
A61N 2005/0661

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**
Benannte Erstreckungsstaaten:
**BA**
Benannte Validierungsstaaten:
**GE KH MA MD TN**

(30) Priorität: 23.02.2024  DE 102024105158

(71) Anmelder: SCHOTT AG
55122 Mainz (DE)

(72) Erfinder:
• THOMAS, Jens Ulrich
55122 Mainz (DE)
• RUSSERT, Hubertus
55122 Mainz (DE)
• SCHULTHEIS, Bernd
55122 Mainz (DE)
• HOPPE, Bernd
55122 Mainz (DE)
• HESSINGER, Carolin
55122 Mainz (DE)
• GÖNÜLLÜ, Yakub
55122 Mainz (DE)

(74) Vertreter: Schott Corporate IP
Hattenbergstraße 10
55122 Mainz (DE)

(54) **BELEUCHTUNGSVORRICHTUNG MIT LICHTVERTEILUNGSELEMENT ZUR BESTRAHLUNG MIT UV- LICHT UND BEHANDLUNGSSYSTEM ZUR BESTRAHLUNG MIT UV-LICHT**

(57) Die Erfindung betrifft eine Beleuchtungsvorrichtung umfassend zumindest eine Lichtquelle, welche Licht einer Wellenlänge im Bereich von 180 nm bis 360 nm emittiert, und ein Lichtverteilungselement mit zwei gegenüberliegenden Seitenflächen. Das Lichtverteilungselement umfasst ein für das eingekoppelte Licht transparentes oder zumindest weitgehend transparentes Material, wobei das Licht der Lichtquelle in das Lichtverteilungselement eingekoppelt wird und aus zumindest einer der beiden Seitenflächen des Lichtverteilungselements austritt. Das Lichtverteilungselement weist Strukturen zur Streuung des eingekoppelten Lichts auf, um das Licht zumindest teilweise so abzulenken, dass es aus zumindest einer der Seitenflächen austritt.

Das Lichtverteilungselement weist zumindest eine Durchgangsöffnung auf, die sich von der einen Seitenfläche des Lichtverteilungselements zur anderen Seitenfläche erstreckt und insbesondere als Durchführung für einen Katheter oder Schlauch ausgebildet ist. Des Weiteren betrifft die Erfindung eine Vorrichtung zur Sterilisation der Haut mit der erfindungsgemäßen Beleuchtungseinrichtung sowie einen Katheter. Der Katheter wird so durch die Durchgangsöffnung geführt, dass das Ende des Katheters, mit welchem der Katheter durch die Haut in den Patienten eingeführt wird, sich auf der Seite des Lichtverteilungselements mit der auskoppelnden Seitenfläche befindet.

Fig. 1

EP 4 606 424 A1

**Beschreibung**

Gebiet der Erfindung

Im Allgemeinen betrifft die Erfindung eine Beleuchtungsvorrichtung, insbesondere für ein medizintechnisches Behandlungssystem, zur Bestrahlung mit UV-Licht. Die Beleuchtungsvorrichtung umfasst hierbei zumindest eine Lichtquelle, welche UV-Licht emittiert, sowie ein Lichtverteilungselement zur Erzeugung einer gleichmäßigen Verteilung des UV-Lichts innerhalb des vorbestimmten Anwendungsbereiches. Insbesondere betrifft die Erfindung eine Beleuchtungsvorrichtung zur Verringerung der Keimdichte auf der Haut bzw. zur Desinfektion der Haut.

In der Medizin werden im Rahmen von diagnostischen Verfahren oder therapeutischen Behandlungen Katheter verwendet. Unter Kathetern werden dabei Röhrchen oder Schläuche verschiedener Durchmesser verstanden, mit denen Hohlorgane wie beispielsweise Harnblase, Magen, Darm aber auch Blutgefäße sondiert, entleert, gefüllt oder gespült werden können. Insbesondere Venenkathetern kommt eine große Bedeutung in der klinischen Praxis zu. Hierbei bieten Venenkatheter, welche auch als zentraler Venenkatheter oder peripherer Venenverweilkatheter bezeichnet werden, die Möglichkeit, dem Patienten über einen relativ langen Zeitraum Medikamente kontinuierlich oder bedarfsmäßig zuzuführen oder beispielsweise zu Diagnosezwecken Blut zu entnehmen. Die Punktierung der Vene durch die Haut stellt jedoch eine Verletzung und somit ein Infektionsrisiko durch Erreger dar, welches mit der Länge der Verweildauer des Katheters steigt.

Um das Infektionsrisiko an der Punktierungsstelle zu senken, wird daher versucht, die Erregeranzahl, d.h. Keimzahl im Bereich der Punktierungsstelle möglichst gering zu halten. So sind aus dem Stand der Technik beispielsweise Vorrichtungen bekannt, welche durch Einstrahlung von UV-Strahlung die Keim- bzw. Erregeranzahl auf der Haut des Patienten im Bereich um die Punktierungsstelle zumindest kurzzeitig verringern.

Aus der DE 10 2009 015 088A sind entsprechende Vorrichtungen bekannt. Vorrichtungen des Typs VisiLED UV Ringlicht der Firma Schott, weisen eine UV-Lichtquelle auf, mit welcher der Bereich um die Punktierungsstelle mit UV-Licht bestrahlt werden kann. Da die Vorrichtungen jedoch relativ groß sind, ist eine kontinuierliche Desinfektion der Punktierungsstelle über die gesamte Verweildauer des Katheters meist nicht möglich oder zumindest nicht praktikabel. Zudem sind diese Geräte recht kostenintensiv. Auch gestaltet sich eine gleichmäßige und zielgenaue Bestrahlung von (Haut) bereichen mit einer Fläche im Bereich von 1 bis 25 cm$^2$ als schwierig. Ein weiterer Nachteil bei der Verwendung entsprechender Vorrichtungen besteht darin, dass bei Verwendung einer punktuellen UV-Quelle nur relativ kleine Bereiche bestrahlt werden können und darüber hinaus Bereiche auf der Haut durch den Katheter abgeschattet werden und somit keine oder nur eine unzureichende UV-Behandlung erfahren. Eine gleichmäßige Verteilung des UV-Lichtes ist dabei auf Grund der geringen Wellenlänge technisch anspruchsvoll, da die meisten Materialien, die für entsprechende optische Elemente verwendet werden, nicht oder nicht ausreichend transparent für UV-Licht mit Wellenlängen unterhalb von 300 nm sind.

Aufgabe der Erfindung

Eine Aufgabe der vorliegenden Erfindung besteht daher darin, eine Beleuchtungsvorrichtung bereit zu stellen, mit der die Keimanzahl auf der Haut, insbesondere an der Punktierungsstelle eines Katheters bzw. in den angrenzenden Bereichen leicht und über Stunden, Tage oder auch Wochen durch Einstrahlung von UV-Licht verringert werden bzw. geringgehalten werden kann. Hierbei soll die Beleuchtungsvorrichtung insbesondere einen Aufbau aufweisen, welcher eine einfache Handhabung sowohl bei der Einführung des Katheters durch medizinisches Fachpersonal als auch beim Verbleib der Beleuchtungsvorrichtung am Patienten während der Verweildauer des Katheters ermöglicht. Eine weitere Aufgabe der Erfindung besteht in der Bereitstellung einer Vorrichtung umfassend einen Katheter und eine Beleuchtungseinrichtung zur Verringerung der Keimzahl auf der Haut im Bereich der Punktierungsstelle.

Die der Erfindung zugrundeliegenden Aufgaben werden bereits durch den Gegenstand der unabhängigen Ansprüche gelöst. Vorteilhafte Ausführungsformen und Weiterbildungen sind Gegenstand der Unteransprüche.

Kurzbeschreibung der Erfindung

Ein Aspekt der Erfindung betrifft eine Beleuchtungseinrichtung zur Abgabe von UV-Licht. Die Beleuchtungseinrichtung ist insbesondere dazu geeignet, als Teil eines Behandlungssystems, eines medizintechnischen Therapiesystems oder eines Diagnosesystems eingesetzt zu werden und umfasst zumindest eine Lichtquelle, welche keimtötend wirksames Licht, insbesondere Licht mit einer Wellenlänge im Bereich von 180 bis 360 nm emittiert. Bei der Lichtquelle handelt es sich somit um eine UV-Lichtquelle. Als UV-Lichtquelle können sowohl konventionelle Lichtquellen wie beispielsweise Quecksilberdampflampen als auch LEDs verwendet werden. Weiterhin umfasst die Beleuchtungsvorrichtung ein scheibenförmiges Lichtverteilungselement, welches das UV-Licht über eine Fläche homogen abstrahlt, wobei das UV-Licht gestreut wird. Das scheibenförmige Lichtverteilungselement weist zwei gegenüberliegende Seitenflächen sowie zumindest eine Fläche zwischen den beiden Seitenflächen auf. Die Fläche, die zwischen den beiden gegenüberliegenden Seitenflächen liegt und in einem Winkel, vorzugsweise in einem Winkel von 90° zu diesen Seitenflächen steht, wird im Rahmen der Offenbarung als Stirnseite oder Stirnfläche bezeichnet. Das Lichtverteilungselement umfasst ein Material, welches für das von der Lichtquelle emittierte Licht transparent oder zumindest weitgehend transparent ist. Insbesondere weist das Material des Lichtverteilungselements für das von der Lichtquelle emittierte Licht für eine Wellenlänge im Bereich von 180 nm bis 360 nm eine Absorption und/oder Streuung von weniger als 10% pro cm, bevorzugt von weniger als 1% pro cm auf. Alternativ oder zusätzlich weist das Material des Lichtverteilungselements eine Dämpfung von weniger als -3 dB/cm, vorzugsweise von weniger als -1 dB/cm für das von der Lichtquelle emittierte Licht auf. Das Lichtverteilungselement kann aus dem UV-transparenten Material bestehen oder das transparente Material umfassen. Eine Ausführungsform sieht vor, dass das Lichtverteilungselement als Hauptkomponente das UV-transparente Material enthält.

Vorzugsweise sind mehr als 50 Gew.-% oder mehr als 70 Gew.-% des Lichtverteilungselements ein UV-transparentes Material. Das UV-transparente Material kann auch als Substrat eingesetzt werden, das beispielsweise mit einer oder mehreren Beschichtungen versehen werden kann. Es hat sich herausgestellt, dass das UV-transparente Material sowohl amorph als auch kristallin oder teilkristallin sein kann. Gemäß einer Ausführungsform wird als UV-transparentes Material Quarzglas, vorzugsweise wasserangereichertes Quarzglas verwendet. Gemäß einer anderen Ausführungsform wird als UV-transparentes Material kristallines $CaF_2$, kristallines $MgF_2$ oder Saphir verwendet.

Das von der Lichtquelle emittierte Licht wird in das Lichtverteilungselement eingekoppelt, durch Strukturen zumindest teilweise abgelenkt und tritt aus zumindest einer der beiden gegenüberliegenden Seitenflächen, bevorzugt aus einer der beiden gegenüberliegenden Seitenflächen aus. Das Lichtverteilungselement ist somit ein Diffusorelement. Die Seitenfläche, aus der das Licht ausgekoppelt wird, wird im Rahmen der Offenbarung auch als auskoppelnde Seitenfläche bezeichnet. Das Lichtverteilungselement weist Strukturen zur Streuung des UV-Lichtes auf. Diese sind vorzugsweise zumindest auf oder an einer der beiden Seitenflächen des Lichtverteilungselements angebracht. Es hat sich als besonders vorteilhaft herausgestellt, wenn zumindest die Seitenfläche des Lichtverteilungselements, durch welche das UV-Licht ausgekoppelt wird, Strukturen zur Streuung des UV-Lichts aufweist.

Das Lichtverteilungselement weist eine Öffnung auf, welche sich von einer Seitenfläche zur anderen, gegenüberliegenden Seitenfläche des Lichtverteilungselements erstreckt. Die Öffnung bildet somit einen nach beiden Enden hin offenen Kanal durch das Lichtverteilungselement. Vorzugsweise ist die Öffnung in einem zentralen Bereich des Lichtverteilungselements angebracht. Die Öffnung wird im Sinne der Offenbarung auch als Durchführung oder Durchführungsöffnung bezeichnet. Insbesondere ist diese Öffnung dazu ausgebildet, als Durchführung für einen Schlauch, insbesondere für einen Katheter, oder eine Venenverweilkanüle, zu fungieren Bei Verwendung der Beleuchtungsvorrichtung mit einem Katheter befindet sich die auskoppelnde Seitenfläche des Lichtverteilungselementes auf der Seite der Katheterspitze. Somit trifft das aus der auskoppelnden Seitenfläche des Lichtverteilungselements austretende UV-Licht auf die Haut im Bereich der Punktierungsstelle. Vorzugsweise wird das UV-Licht über die gesamte auskoppelnde Seitenfläche des Lichtverteilungselements abgestrahlt. Durch diese Anordnung wird eine Abschattung durch den Schatten des Katheters minimiert und eine homogene Bestrahlung ermöglicht.

Neben der Verwendung der Beleuchtungsvorrichtung im Zusammenhang mit Kathedern oder ähnlichen in oder am Körper eines Patienten verbleibenden Therapiesystemen, ist eine derartige Beleuchtungsvorrichtung mit einem Lichtverteilungselement zur Verteilung von UV-Licht allgemein zum Einsatz in einem medizintechnischen Behandlungssystem geeignet, beispielsweise wenn zur Diagnose oder Behandlung bzw. Vorbereitungen dazu die Applikation von UV-Licht insbesondere zur Keimreduktion oder Desinfektion erfolgen soll oder auch wenn vor, während und/ oder nach einer Behandlung die Applikation von UV Licht notwendig, sinnvoll oder erwünscht ist. Damit das aus der auskoppelnden Seitenfläche des Lichtverteilungselements abgestrahlte UV-Licht zu einer Keimabtötung führt und somit eine desinfizierende bzw. keimabtötende Wirkung entfaltet, wird eine Mindestlichtintensität des auf der Hautoberfläche auftreffenden UV-Lichts benötigt. Gleichzeitig darf die Intensität des auf die Haut auftreffenden UV-Lichts nicht zu hoch sein, um Zellschädigungen der Haut zu vermeiden. Gemäß einer Ausführungsform beträgt die Lichtintensität in einem Abstand von 5 bis 20 mm zur auskoppelnden Seitenfläche des Lichtverteilungselements daher 1 bis 50 $\mu W/mm^2$, bevorzugt 2 bis 20 $\mu W/mm^2$ und besonders bevorzugt 5 bis 15 $\mu W/mm^2$. Alternativ oder zusätzlich weist das aus der auskoppelnden Seitenfläche austretende Licht eine integrale Lichtleistung von zumindest 1 mW, bevorzugt von zumindest 2 mW auf. Unter der integralen Lichtleistung wird dabei die Energie im Abstand von 5 bis 20 mm zur auskoppelnden Seitenfläche und integriert über die gesamte Beleuchtungsfläche des abgegebenen Lichtes verstanden.

Die entsprechende Lichtintensität sollte dabei über den gesamten bestrahlten Bereich bzw. auf der gesamten Fläche unterhalb der auskoppelnden Seitenfläche möglichst homogen verteilt sein. Hierdurch wird sichergestellt, dass im gesamten bestrahlten Bereich eine desinfizierende Wirkung auftritt. Die desinfizierende bzw. keimabtötende Wirkung ist dabei vorzugsweise nahe der Punktierungsstelle des Katheters am größten. Die homogene Intensitätsverteilung wird dabei insbesondere dadurch erreicht, dass das UV-Licht nicht nur punktuell von oben abgegeben wird, sondern durch den Einsatz des Lichtverteilungselements über eine relativ große Fläche abgegeben wird. Zudem erfolgt die Abstrahlung relativ nahe an der zu bestrahlenden Hautfläche. Durch diese Anordnung wird dabei der Schattenwurf des Katheters gegenüber einer punktuellen Abstrahlung deutlich reduziert.

Gemäß einer Ausführungsform ist die Bestrahlungsintensität des vom Lichtverteilungselement ausgekoppelten UV-Lichts daher auf einer Fläche, die unterhalb des Lichtverteilungselementes angeordnet ist, so homogenisiert, dass entlang einer kreisförmigen Begrenzungslinie in einem vorgegebenen Abstand vom Mittelpunkt der Öffnung bis maximal 2 cm von diesem Mittelpunkt das Verhältnis des Maximums der Bestrahlungsintensität und des Minimums der Bestrahlungsintensität ein Verhältnis von höchstens 3, vorzugsweise von höchstens 2 beträgt und die Bestrahlungsintensität ihr Maximum in einem Bereich aufweist, der höchstens 1,5 cm, vorzugsweise höchstens 1 cm vom Mittelpunkt der Öffnung entfernt ist.

Eine Ausführungsform sieht vor, dass der Bereich der Seitenfläche, aus dem das UV-Licht ausgekoppelt wird, eine Fläche im Bereich von 1 bis 25 cm$^2$, bevorzugt im Bereich von 1 bis 20 cm$^2$, besonders bevorzugt im Bereich von 1 bis 8 cm$^2$ und ganz besonders bevorzugt im Bereich von 1 bis 4 cm$^2$ aufweist.

Gemäß einer Ausführungsform ist das Lichtverteilungselement kreisförmig oder ellipsoid geformt. Für eine homogene Lichtausbreitung kann auch vorteilhaft sein, wenn die äußere Form des Lichtverteilungselementes nicht vollständig kreisförmig ist. So sieht eine Weiterbildung vor, dass das Lichtverteilungselement kreisförmige oder ellipsoide Anteile aufweist, jedoch keinen vollständigen Kreis bzw. keine vollständige Ellipse bildet. So kann das Lichtverteilungselement insbesondere D-förmig sein, d.h. kreisförmig mit einem fehlenden Kreissegment.

Alternativ ist das Lichtverteilungselement als polygonale Scheibe, vorzugsweise als Polygon mit zumindest 4, besonders bevorzugt zumindest 5 Ecken, geformt. Hierdurch kann ebenfalls eine zirkulare Lichtverteilung vermieden werden, gleichzeitig weist das Lichtverteilungselement jedoch eine hohe Symmetrie auf, so dass bei der Verwendung des Lichtverteilungselements eine Ausrichtung des Lichtverteilungselements weitgehend vernachlässigt werden kann.

Als besonders vorteilhaft haben sich hierbei Lichtverteilungselemente mit einer maximalen Querabmessung im Bereich von 1 bis 8 cm, vorzugsweise im Bereich von 2 bis 6 cm herausgestellt. Eine weitere Ausführungsform sieht vor, dass das Lichtverteilungselement eine maximale Querabmessung im Bereich von 1 bis 4 cm, vorzugsweise im Bereich von 1,5 bis 3 cm aufweist. Unter der maximalen Querabmessung wird der maximale Abstand zweier Kanten bzw. Punkte auf der Kante derselben Seitenfläche verstanden, wobei die Durchgangsöffnung bei der Bestimmung dieses Abstandes nicht berücksichtigt wird. Bei kreisförmigen Lichtverteilungselementen entspricht die maximale Querabmessung dem Kreisdurchmesser. Insbesondere bei Verwendung der Beleuchtungseinrichtung in Verbindung mit einem Katheter, wie beispielsweise einem Venenkatheter oder einem vergleichbar dimensionierten Katheter, haben sich die oben beschriebenen Abmessungen als vorteilhaft herausgestellt. So ist durch die Größe des Lichtverteilungselementes einerseits eine homogene Bestrahlung einer ausreichend großen Fläche gewährleistet, während die Abmessungen gleichzeitig klein genug für ein unkompliziertes Handling, beispielsweise bei Verbleiben der Beleuchtungseinrichtung am Katheter über dessen gesamte Verweildauer im Patienten sind.

Eine Ausführungsform sieht vor, dass die Lichtquelle UV-Licht einer Wellenlänge im Bereich von 180 nm bis 250 nm insbesondere im Bereich von 200 bis 230 nm emittiert. Gemäß einer anderen Ausführungsform emittiert die Lichtquelle UV-Licht mit einer Wellenlänge im Bereich von 250 bis 300 nm. Insbesondere in diesen Wellenlängenbereichen ist bei ausreichender Lichtintensität eine desinfizierende bzw. keimtötende Wirkung des emittierten Lichts gewährleistet. Gleichzeitig ist bei diesen Wellenlängen die Eindringtiefe, beispielsweise in die Haut, relativ gering, was in Hinblick auf die gewünschte Desinfektion der Hautoberfläche vorteilhaft ist.

Das Lichtverteilungselement umfasst ein Material, dass für das von der Lichtquelle emittierte Licht transparent oder weitgehend transparent ist. Hierbei können sowohl amorphe als auch kristalline Materialien eingesetzt werden. Gemäß einer Ausführungsform umfasst oder besteht das Lichtverteilungselement aus UV-transparentem $SiO_2$, Saphir $CaF_2$ oder $MgF_2$. Als besonders vorteilhaft hat sich die Verwendung von wasserhaltigem $SiO_2$ herausgestellt. Durch den Wassergehalt bzw. die zusätzlichen Hydroxylgruppen weist das $SiO_2$ eine besonders geringe Absorption im UV-Bereich, insbesondere auch für niedrige Wellenlängen auf.

Das Lichtverteilungselement fungiert als Diffusor. Es hat sich herausgestellt, dass eine besonders homogene Lichtverteilung erzielt werden kann, wenn eine der beiden Seitenflächen des Lichtverteilungselementes zumindest in Teilbereichen eine Rauheit RMS im Bereich von 1 bis 400 nm, bevorzugt im Bereich von 10 bis 200 nm und besonders bevorzugt im Bereich von 50 bis 150 nm aufweist. Eine Ausführungsform sieht vor, dass die Seitenfläche, aus der das Licht ausgekoppelt wird, eine entsprechende Rauheit aufweist. Hierbei kann die Seitenfläche eine einheitliche Rauheit aufweisen. Die RMS-Rauheit ist dabei die sogenannte quadratische Rauheit (root-mean-squared roughness: Wurzel des Mittelquadrates) und wird aus dem Mittel der Abweichungsquadrate berechnet und entspricht dem "quadratischen Mittel" der Messwerte über eine Messstrecke I. Diese beispielsweise mittels optischer Verfahren, wie Weißlichtinterferometrie oder konfokaler mikroskopischer Verfahren ermittelt werden.

Alternativ ist ein Lichtverteilungselement vorgesehen, bei dem die Seitenfläche mit erhöhter Rauheit, vorzugsweise die auskoppelnde Seitenfläche, Teilbereiche mit unterschiedlicher Rauheit aufweist. Somit unterscheidet sich bei dieser Ausführungsform die Rauheit lokal. Die Rauheit kann hierbei einen Gradientenverlauf aufweisen. Hierbei kann die RMS- Rauheit beispielsweise einen Gradienten von 0,2 nm bis 150 nm aufweisen. Gemäß einer Ausführungsform weist die Seitenfläche mit erhöhter Rauheit eine RMS-Rauheit im Bereich von 0,2 bis 100 nm, bevorzugt im Bereich von 0,3 bis 80 nm auf. Somit kann die entsprechende Seitenfläche sowohl sehr glatte oder sogar polierte Bereiche als auch angeraute Bereiche, d.h. Bereiche mit einer höheren Rauheit, aufweisen. Vorzugsweise nimmt die Rauheit mit zunehmenden Abstand von der Stelle, an der das UV-Licht in das Lichtverteilungselement eingekoppelt wird, zu. Hierdurch wird erreicht, dass Bereiche, die weiter von der Einkoppelstelle entfernt sind, eine höhere Auskopplungsrate aufweisen als die weniger rauen Bereiche nahe der Einkoppelstelle. Als Einkoppelstelle wird im Sinne der Offenbarung insbesondere der Bereich des Lichtverteilungselements verstanden, an dem das von der Lichtquelle emittierte Licht in das Lichtverteilungselement eingekoppelt wird. Durch diesen Effekt unterschiedlicher Rauheit bzw. eines Gradienten der Rauheiten können Verluste, die bei der Lichtweiterleitung innerhalb des Lichtverteilungselements beispielsweise durch Streuung und/oder Auskopplung auftreten, ausgeglichen und somit ein homogenes Abstrahlverhalten über die gesamte auskoppelnde Seitenfläche des Lichtverteilungselements erzielt werden.

Ein vergleichbarer Effekt kann alternativ oder auch zusätzlich durch Mikrostrukturierungen auf einer der beiden Seitenflächen des Lichtverteilungselements, vorzugsweise auf der auskoppelnden Seitenfläche des Lichtverteilungselements, erzielt werden. Die entsprechenden Mikrostrukturen können durch verschiedene Verfahren gebildet werden. Insbesondere Verfahren wie beispielsweise Laserablation, Laserstrukturierung oder Heißformungsprozesse (z.B. Pressen, Prägen), sowie auch nass- oder trockenchemische Ätzprozesse mit ggf. vorgeschalteten Lithographie-Prozessen, oder auch vorgeschalteten Laserstrukturierungsprozessen oder Laserprozessen, die es erlauben das Material des Substrats so zu modifizieren, dass dies gezielt oder vorbestimmbar strukturiert bzw. geätzt werden kann, sind hierzu geeignet. Auch eine Mikrostrukturierung einer oder beider Seitenflächen durch Sandstrahlen ist möglich. Derartige Mikrostrukturierungen können zusätzlich zur o.g. Rauigkeit dazu beitragen, dass das Licht von der Einkoppelstelle kommend homogener verteilt werden kann. Eine Ausführungsform sieht vor, dass die Mikrostrukturen durch eine strukturierte Beschichtung, beispielsweise durch eine strukturierte Silberbeschichtung, gebildet werden. Gemäß einer Ausführungsform der Erfindung weist das Lichtverteilungselement in lokal begrenzten Bereichen der Seitenfläche eine oder mehrere Mikrostrukturen auf, mit denen die lokale Intensität des ausgekoppelten Lichts bzw. der Anteil des ausgekoppelten Lichts eingestellt werden kann. Dies kann durch Lichtstreuung erfolgen. Mikrostrukturen werden im Sinne der Erfindung nicht auf Strukturen im Mikrometerbereich beschränkt verstanden, sondern auch größere oder gröbere Strukturen und insbesondere kleinere oder feinere Strukturen im Sub-Mikrometer- oder Nanometerbereich.

Gemäß einer Ausführungsform weist eine der beiden Seitenflächen des Lichtverteilungselements eine geringere Rauheit auf als die andere Seitenfläche. Insbesondere weist die Seitenfläche mit der geringeren Rauheit eine Rauheit RMS von weniger als 0,5 nm, bevorzugt weniger als 0,2 nm und besonders bevorzugt weniger als 0,1 nm auf.

Vorzugsweise ist die Rauheit der Seitenfläche, welche der auskoppelnden Seitenfläche gegenüberliegt, geringer als die Rauheit der auskoppelnden Seitenfläche. Hierdurch wird eine möglichst vollständige Reflexion des Lichtes an dieser Seitenfläche begünstigt. Eine möglichst vollständige Reflexion bzw. sogar eine Totalreflexion an dieser Seitenfläche ist vorteilhaft, da somit annähernd das gesamte eingekoppelte Licht durch die auskoppelnde Seitenfläche abgegeben wird und kein bzw. nur ein sehr geringer Intensitätsverlust des eingekoppelten Lichtes durch die gegenüberliegende Seitenfläche erfolgt. Dieser Intensitätsverlust kann weiter minimiert werden, wenn zumindest eine der Seitenflächen des Lichtverteilungselementes eine für die Wellenlänge des verwendeten UV-Lichtes hoch reflektierende Beschichtung, bevorzugt eine reflektierende Aluminiumbeschichtung oder Silberbeschichtung, aufweist. Grundsätzlich könnten auch mehrschichtige dielektrische Reflektorschichten zum Einsatz kommen, wenn diese derart von der Schichtfolge ausgelegt sind, dass der üblicherweise sehr hohe Reflektionsgrad auch über große Reflektions-Winkelbereiche (> 45° ggü. der Senkrechten zur Seitenfläche) ausgelegt ist. Hierbei kann sowohl die auskoppelnde Seitenfläche als auch die ihr gegenüberliegende Seitenfläche eine entsprechende Beschichtung aufweisen.

Das Lichtverteilungselement weist gemäß einer Ausführungsform ein einstückiges, vorzugsweise monolithisch ausgebildetes Substrat aus einem für das emittierte Licht der Lichtquelle transparenten Material auf. Dies ermöglicht insbesondere die Bereitstellung des Lichtverteilungselements durch ein Herstellungsverfahren mit wenigen Fertigungsschritten. So kann beispielsweise das Substrat in einem Schritt geformt und nachfolgend beschichtet werden. Dies ist insbesondere in Hinblick auf kostengünstige Herstellungsverfahren vorteilhaft und ermöglicht darüber hinaus ein kompaktes, platzsparendes Design des Lichtverteilungselements. Alternativ kann das Lichtverteilungselement jedoch auch aus einem mehrteiligen bzw. mehrteiligen Substrat aufgebaut sein. Mit anderen Worten das Lichtverteilungselement kann aus oder in einer Scheibe eines Subtratmaterials ausgebildet oder auch aus oder mit zumindest zwei Scheiben eines oder mehrerer Substratmaterialien ausgebildet sein. Im Falle einer mehrteiligen Ausführung umfasst das Lichtleitelement ggf. zwischen den zumindest zwei Scheiben Abstandhalter, die zumindest teil- oder abschnittsweise auf oder am Außenradius der Scheiben angeordnet sind und zum Beispiel als Ring oder Ringsegmente ausgebildet sein können. Weiterhin ist es im Falle der mehrteiligen Ausführung denkbar, dass zumindest das Material auf der Auskoppelseite transparent im relevanten Wellenlängenbereich ist. Die übrigen Materialien können, aber müssen ggf. diese Eigenschaft nicht aufweisen.

Eine Ausführungsform sieht vor, dass das Lichtverteilungselement als kreisförmige Scheibe mit einem maximalen Durchmesser im Bereich von 20 bis 30 mm und einer Dicke im Bereich von 0,4 bis 1,5 mm ausgebildet ist. In dieser Ausführungsform ist die Öffnung zur Durchführung eines Schlauchs oder Katheters bevorzugt mittig im Lichtverteilungselement angeordnet und weist einen Durchmesser im Bereich von 2 bis 6 mm auf. Vorzugsweise ist die Öffnung kreisförmig oder elliptisch ausgebildet.

Die Wandungen der Öffnung können parallel zur Oberflächennormalen verlaufen, die Öffnung verläuft somit weitgehend senkrecht von der Oberfläche einer Seitenfläche zur Oberfläche der gegenüberliegenden Seitenfläche.

Es sind auch Ausführungsformen möglich, bei denen die Öffnung zur Durchführung eines Schlauchs oder Katheters als Schlitz ausgebildet ist und sich der Schlitz von einem äußeren Randbereich des Lichtverteilungselements in einen zentralen Bereich erstreckt. Diese Ausgestaltungen weisen zwar den Nachteil einer geringeren Homogenität des abgestrahlten Lichts auf, ermöglichen jedoch, die Beleuchtungsvorrichtung auch bei einem bereits am Patienten gelegten Katheter hinzuzufügen oder auch zu entfernen. In einer Weiterbildung kann das Lichtverteilungselement mehrteilig ausgebildet sein. Hierbei weist ein Teilstück des Lichtverteilungselements den Schlitz zur Durchführung auf, der nach der Durchführung des Katheters durch den Schlitz mit einem weiteren Teilstück verschlossen oder überdeckt werden kann. Hierdurch kann auch in dieser Weiterbildung eine hohe Homogenität des ausgekoppelten Lichts erzielt werden.

Bei der Platzierung der Beleuchtungsvorrichtung am Patienten ist es vorteilhaft, wenn die auskoppelnde Seitenfläche einen räumlichen Abstand zur Haut des Patienten aufweist. Eine Weiterbildung der Erfindung sieht daher vor, dass das Lichtverteilungselement in einem umlaufenden Halter fixiert ist, wobei zumindest ein Bereich des Halters über die auskoppelnde Seitenfläche des Lichtverteilungselements hinausragt. Dieser Bereich des Halters dient als Abstandhalter zwischen Lichtverteilungselement und der Haut des Patienten. Vorzugsweise weist dieser Bereich der Halterung eine Höhe im Bereich von 5 bis 15 mm auf. Vorzugsweise umfasst der Halter ein organisches Polymer, insbesondere ein Polysiloxan (z.B. ein Silikon) oder ein Polycarbonat, wobei das organische Polymer eine genügende UV-Stabilität aufweisen sollte.

Gemäß einer Ausführungsform weist das Lichtverteilungselement eine Öffnung mit schrägen Wandungen auf. In dieser Ausführungsform verlaufen die Wandungen der Öffnung vorzugsweise mit einem Winkel im Bereich von 5 bis 45° zur Oberflächennormalen. Dies ist insbesondere bei Positionierung der Beleuchtungseinrichtung an schwer zugänglichen Stellen am Patienten und/oder bei Kathetern, die in einem flachen Winkel gelegt werden, vorteilhaft. Eine solche schräge Wandung der Öffnung hätte zudem die Doppelfunktion, den Katheter in einem definierten Winkel zur Haut zu führen, wenn das Lichtverteilungselement durch das Halterungselement parallel zur Hautoberfläche ausgerichtet ist.

Eine Ausführungsform der Erfindung sieht vor, dass das Licht der Lichtquelle über zumindest einen Lichtleiter in das Lichtverteilungselement eingekoppelt wird. Diese Ausführungsform bietet den Vorteil, dass dadurch Lichtquelle und Lichtverteilungselement räumlich voneinander entkoppelt bzw. getrennt werden können. Hierdurch kann der Teil der Beleuchtungsvorrichtung, welcher unmittelbar am Patienten angebracht wird, möglichst platzsparend ausgebildet werden. Es ist dabei auch möglich, das Licht der Lichtquelle über mehrere Lichtleiter in das Lichtverteilungselement einzukoppeln, um so bereits eine homogenere Einkopplung über das gesamte Lichtverteilungselement zu erreichen. Der oder die Lichtleiter können mit der Oberfläche oder in der bzw. durch die Stirnfläche des Lichtverteilungselements verspleißt oder verklebt werden. Eine Ausführungsform sieht vor, dass der oder die Lichtleiter durch ein Glas- oder Kunststofflot bzw. einem polymerbasierten Kleber mit dem Lichtverteilungselement verbunden werden.

Gemäß einer Weiterbildung wird das Licht der Lichtquelle über zumindest einen Lichtleiter in das Lichtverteilungselement eingekoppelt, wobei der Lichtleiter an seinem distalen Ende, welches mit dem Lichtverteilungselement verbunden ist, eine Numerische Apertur NA > 0,1, bevorzugt größer 0,2 aufweist. Hierdurch wird bereits der Lichtkegel des Einkopplungsbereichs gespreizt bzw. der Lichtkegel, der vom Lichtleiter ausgeht, geweitet. Der ausgehende Lichtkegel des distalen Lichtleiterendes kann zudem beispielsweise durch Anschrägen oder Aufrauen der Lichtleiterfaser weiter vergrößert werden.

Zur Einkopplung des Lichtes ist das distale Ende des Lichtleiters gemäß einer Ausführungsform mit der Seitenfläche des Lichtverteilungselements, welche der auskoppelnden Seitenfläche gegenüberliegt, verbunden.

Alternativ kann der Lichtleiter über eine Stirnseite des Lichtverteilungselements verbunden sein. Als Stirnseite wird beispielsweise bei einem scheibenförmigen Lichtverteilungselement die umlaufende Fläche zwischen den beiden Seitenflächen verstanden.

Eine Weiterbildung sieht vor, dass die Einkopplung des Lichtes über eine schräge Fläche des Lichtverteilungselements erfolgt. Die schräge Fläche kann insbesondere durch einen Teilbereich der Stirnseite des Lichtverteilungselements gebildet werden.

Vorzugsweise weist die schräge Fläche, an der das Licht eingekoppelt wird, einen Winkel a zu den Seitenflächen des Lichtverteilungselements im Bereich von 30 bis 50° auf. Die schräge Einkopplungsfläche kann zudem eine Rauheit RMS von > 1nm, bevorzugt >2 nm, besonders bevorzugt > 10 nm und ganz besonders bevorzugt > 50 nm aufweisen.

Gemäß einer anderen Ausführungsform erfolgt die Einkopplung des Lichts über die Kantenfläche, welche die Durchführungsöffnung für einen Schlauch oder Katheter bildet. Durch diese Einkopplung des Lichts mit tangentialer Komponente zum Radius entlang dieser Öffnung kann eine Schattenbildung durch den Schlauch oder Katheter vermieden oder zumindest reduziert werden. Dies ist vorteilhaft, um ggf. auch in abgeschatteten Bereichen sicherzustellen, dass die Intensität des UV-Lichts ausreichend hoch ist, um eine effektive Sterilisation bzw. Desinfektion im abgeschatteten Bereich zu gewährleisten.

Eine andere Ausführungsform sieht vor, dass das Licht der Lichtquelle direkt von der Lichtquelle in eine Seitenfläche des Lichtverteilungselements eingekoppelt wird. Unter einer direkten Einkopplung wird insbesondere verstanden, dass das von der Lichtquelle emittierte Licht von der Lichtquelle ausgehend in das Lichtverteilungselement strahlt und nicht über einen Lichtleiter zum Lichtverteilungselement geleitet wird. Vorzugsweise erfolgt die direkte Einkopplung durch die Seitenfläche des Lichtverteilungselements, die der Seitenfläche, aus der das Licht ausgekoppelt wird, gegenüberliegt. Die Lichtquelle ist mit dieser Seitenfläche fest verbunden. Dies kann beispielsweise durch eine Halterung oder eine Klebeverbindung erfolgen. Vorzugsweise werden als Lichtquellen UV-LEDs verwendet. Auch ist der Einsatz von UV-Laserdioden denkbar. Eine besonders gleichmäßige Einkopplung des Lichtes über das gesamte Lichtverteilungselement kann erfolgen, wenn die Beleuchtungseinrichtung mehrere, mit der Seitenfläche des Lichtverteilungselements form- oder stoffschlüssig verbundene Lichtquellen aufweist.

Ein weiterer Aspekt der Erfindung betrifft eine Vorrichtung zur Sterilisation umfassend die erfindungsgemäße Beleuchtungsvorrichtung sowie einen Katheter, wobei der Katheter so durch die Öffnung des Lichtverteilungselements geführt wird, dass die Spitze des Katheters bzw. das Ende des Katheters, mit welchem der Katheter in den Körper des Patienten eingeführt wird, sich auf der auskoppelnden Seitenfläche des Lichtverteilungselements befindet. Die Beleuchtungsvorrichtung umfasst eine umlaufende Halterung, welche zumindest auf der auskoppelnden Seitenfläche des Lichtverteilungselements über das Lichtverteilungselement herausragt. Die Halterung fungiert somit als Abstandhalter zwischen der auskoppelnden Seitenfläche des Lichtverteilungselements und der Haut des Patienten.

Detaillierte Beschreibung der Erfindung

Nachfolgend wird die Erfindung anhand von Ausführungsbeispielen sowie den Figuren 1 bis 20 näher beschrieben. Es zeigen:

Fig. 1          eine Seitenansicht einer schematischen Darstellung der Beleuchtungsvorrichtung gemäß einem ersten Ausführungsbeispiel,

Fig. 2          das in Fig. 1 gezeigte Ausführungsbeispiel in Aufsicht,

Fig. 3          die schematische Darstellung eines zweiten Ausführungsbeispiels der Beleuchtungsvorrichtung in Aufsicht,

Fig. 4 bis 6    schematische Darstellungen des Lichtverteilungselements verschiedener Ausführungsformen im Querschnitt,

Fig. 7          eine schematische Darstellung einer Ausführungsform eines mehrteiligen Lichtverteilungselements im Querschnitt,

Fig. 8          eine schematische Darstellung einer Ausführungsform eines einstückigen Lichtverteilungselements mit schräger Einkoppelfläche im Querschnitt,

Fig. 9          eine schematische Darstellung einer Ausführungsform eines Lichtverteilungselements mit schräger Durchgangsöffnung im Querschnitt,

Fig. 10         eine schematische Darstellung einer Ausführungsform eines Lichtverteilungselements mit einer auskoppelnden Seitenfläche mit lokal unterschiedlichen Rauheiten in Aufsicht auf die auskoppelnde Seitenfläche,

Fig. 11         eine schematische Darstellung eines ersten Ausführungsbeispiels einer Vorrichtung zur Sterilisation der Haut mit Einkopplung über eine Seitenfläche des Lichtverteilungselements im Querschnitt,

Fig. 12         eine schematische Darstellung eines zweiten Ausführungsbeispiels einer Vorrichtung zur Sterilisation der Haut mit Einkopplung über eine Stirnseite des Lichtverteilungselements im Querschnitt,

Fig. 13         eine schematische Darstellung eines Querschnitts eines dritten Ausführungsbeispiels einer Vorrichtung zur Sterilisation der Haut, bei der die Lichtquellen stoffschlüssig mit dem Lichtverteilungselement verbunden sind,

Fig. 14         die schematische Darstellung der in Fig. 13 gezeigten Ausführungsform in Aufsicht,

Fig. 15         eine schematische Darstellung des Aufbaus zur Bestimmung der Homogenität des abgestrahlten Lichts,

Fig. 16         eine schematische Darstellung der Durchmesser d0 und d1 zur Bestimmung der Homogenität des abgestrahlten Lichts,

Fig. 17         eine schematische Darstellung zur Ermittlung des minimalen abgeschatteten Bereichs,

Fig. 18         eine schematische Darstellung zur Ermittlung des maximalen abgeschatteten Bereichs und

Fig. 19,20      schematische Darstellungen zweier Ausführungsbeispiele einer Beleuchtungsvorrichtung mit D-förmigen bzw. polygonalen Lichtverteilungselementen.

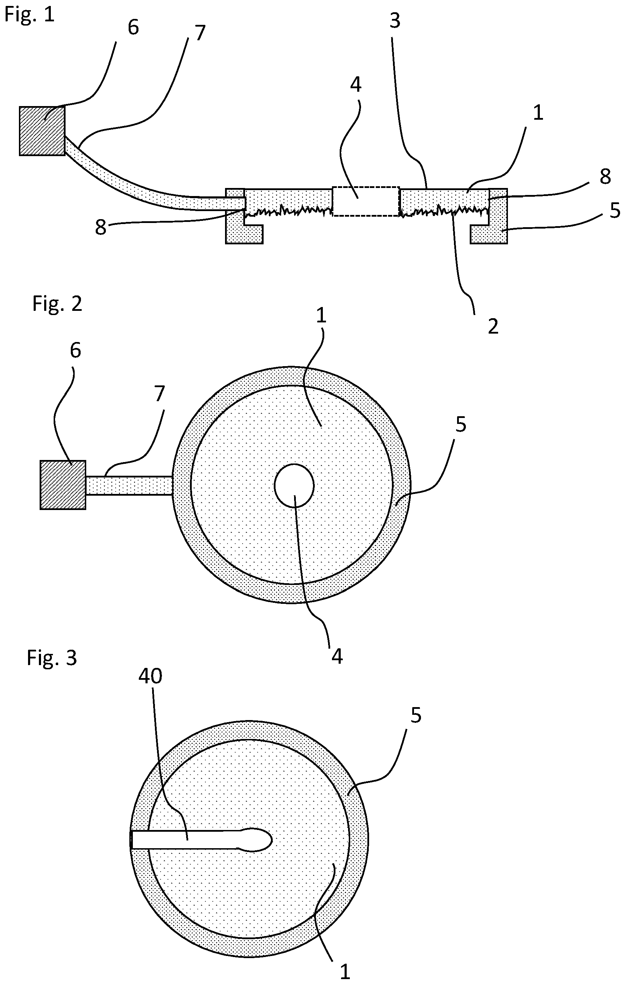

In Fig. 1 ist ein schematischer Querschnitt durch eine Beleuchtungsvorrichtung gemäß einem Ausführungsbeispiel dargestellt. In diesem Ausführungsbeispiel umfasst die Beleuchtungsvorrichtung eine Lichtquelle 6, die UV-Licht mit einer Wellenlänge im Bereich von 180 bis 250 nm emittiert, sowie ein Lichtverteilungselement 1. Das Lichtverteilungselement 1 umfasst ein Material, welches transparent oder zumindest weitgehend transparent für das von der Lichtquelle 6 emittierte Licht ist. Insbesondere weist das Material des Lichtverteilungselement 1 eine Dämpfung für das von der Lichtquelle 6 emittierte Licht von weniger als -3 dB/cm auf. Lichtverteilungselement 1 und Lichtquelle 6 sind über einen Lichtleiter 7 miteinander verbunden. Durch Verwendung eines Lichtleiters 7 sind Lichtquelle 6 und Lichtverteilungselement 1 räumlich voneinander getrennt. Die Länge des Lichtleiters 7 kann an die jeweiligen Begebenheiten angepasst werden und ist in den Figuren nicht maßstabsgerecht abgebildet. Bei dem Lichtleiter 7 handelt es sich insbesondere um eine Quarzglasfaser. Das Lichtverteilungselement 1 ist als Scheibe mit den beiden Seitenflächen 3 und 2 sowie einer umlaufenden Stirnseite 8 ausgebildet. Die Einkopplung des von der Lichtquelle 6 emittierten UV-Lichts erfolgt an der Stirnseite 8 des Lichtverteilungselements 1. Hierzu ist der Lichtleiter 7 mit, an oder in der Stirnseite 8 des Lichtverteilungselements 1 verspleißt, verklebt oder so fixiert, dass das distale Faserende bündig im Kontakt mit der Stirnseite ist. Die Seitenfläche 2 des Lichtverteilungselements 1 ist als auskoppelnde Seitenfläche ausgebildet und weist eine Strukturierung in Form einer aufgerauten Oberfläche auf. Durch die auf der Seitenfläche 2 aufgebrachte Strukturierung wird das ausgekoppelte Licht gestreut, so dass die gesamte Fläche unter dem Lichtverteilungselement 1 homogen oder zumindest weitgehend homogen bestrahlt wird. Das Lichtverteilungselement 1 weist eine Durchführungsöffnung 4 zur Durchführung eines Schlauchs oder Katheters auf. Die Durchführungsöffnung 4 erstreckt sich durch das Material des Lichtverteilungselements 1 von der Seitenfläche 2 zur Seitenfläche 3 und bildet somit einen offenen Kanal durch das Lichtverteilungselement 1. In dem in Fig. 1 dargestellten Ausführungsbeispiel ist die Öffnung 4 mittig im Lichtverteilungselement 1 angeordnet und weist senkrechte Wandungen auf. Das Lichtverteilungselement 1 wird von einem umlaufenden Halterungselement 5, im Folgenden auch als Halterung bezeichnet, gehalten. Die Halterung 5 erstreckt sich auch unterhalb der auskoppelnden Seitenfläche 2 und weist im gezeigten Ausführungsbeispiel ein L-förmiges Profil auf. Im Bereich der Einkoppelstelle weist die Halterung 5 eine Öffnung für den Lichtleiter 7 auf.

Fig. 2 zeigt das oben beschriebene Ausführungsbeispiel in Aufsicht. Es wird deutlich, dass in diesem Ausführungsbeispiel das Lichtverteilungselement 1 als kreisförmige Scheibe ausgebildet ist und die Durchführungsöffnung 4 im Mittelpunkt dieser Scheibe liegt.

Fig. 3 zeigt ein weiteres Ausführungsbeispiel eines Lichtverteilungselements 1 samt Halterung 5. Auch hier ist das Lichtverteilungselement 1 eine kreisförmige Scheibe, die Öffnung zur Durchführung eines Katheters ist jedoch als Schlitz 40 ausgebildet. Der Schlitz 40 erstreckt sich hierbei von einem Randbereich des Lichtverteilungselements 1 bis zu dessen Mittelpunkt. Durch diese Ausgestaltung der Durchgangsöffnung als Schlitz 40 kann das Lichtverteilungselement 1 bzw. die gesamte Beleuchtungsvorrichtung auch nach dem Legen des Katheters hinzugefügt oder entfernt werden.

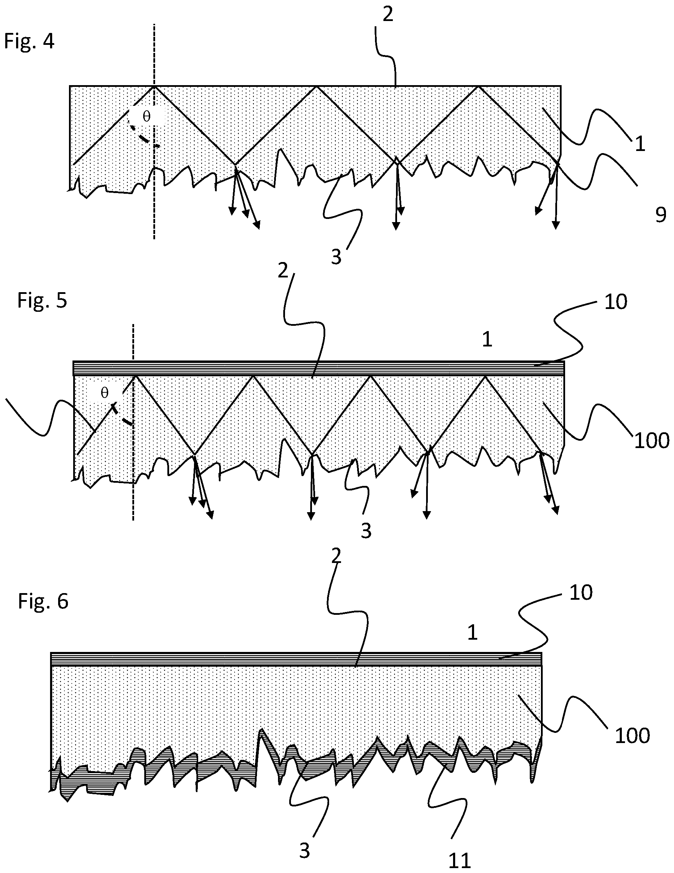

In den Fig. 4 bis 6 sind Querschnitte durch das Lichtverteilungselement 1 verschiedener Ausführungsbeispiele schematisch dargestellt. In Fig. 4 wird ein einstückiges Lichtverteilungselement 1 dargestellt, welches eine glatte Seitenfläche 2 und eine strukturierte Seitenfläche 3 aufweist. Die Seitenfläche 3 weist eine höhere Rauheit RMS auf als die ihr gegenüberliegende Seitenfläche 2 und ist die auskoppelnde Seitenfläche des Lichtverteilungselements. Der Linie 9 symbolisiert den Strahlengang des eingekoppelten Lichts, durch die Pfeile wird das ausgekoppelte Licht dargestellt. Da das Material des Lichtverteilungselements 1 für das eingekoppelte Licht transparent ist, breitet dieses sich innerhalb des Lichtverteilungselements 1 aus. Wenn das Material des Lichtverteilungselementes die Brechzahl n besitzt, werden Lichtstrahlen mit Auftreffwinkel $\theta$, innerhalb des Lichtverteilungselementes an den Seitenflächen total reflektiert, wenn die Auftreffwinkel größer als der kritische Winkel $\theta_c = \arcsin(1/n)$ sind. Das Licht 9 wird so an der Seitenfläche 2 des Lichtverteilungselements 1 reflektiert und trifft auf die aufgeraute Seitenfläche 3. Durch die raue Struktur der Seitenfläche 3 erfolgt keine vollständige Reflexion des Lichts 9 zurück ins Innere des Lichtverteilungselements 1, sondern ein Teil des Lichts wird aus der Seitenfläche 3 ausgekoppelt und dabei gestreut. Vorzugsweise wird die Rauheit bzw. Körnung der angerauten Seitenfläche so eingestellt, dass das Licht überwiegend vorwärts gestreut wird und so das Lichtverteilungselement 1 durch die auskoppelnde Seitenfläche 2 verlässt. Das Lichtverteilungselement 1 fungiert somit auch als Diffusor.

Fig. 5 zeigt eine weitere Ausführungsform eines Lichtverteilungselements 1. Hier umfasst das Lichtverteilungselement 1 neben einem Substrat 100 aus einem für das Licht 9 transparenten Material eine reflektierende Beschichtung 10. Die reflektierende Beschichtung 10 ist auf der Seitenfläche 2, d.h. auf der Seitenfläche, die nicht auskoppelt, aufgebracht. Durch die Beschichtung 10 kann die Reflexion des Lichtes innerhalb des Lichtverteilungselements 1 verbessert und so Lichtverluste minimiert werden. Abhängig von der Güte bzw. Beschaffenheit der Beschichtung 10 kann die Reflektion im Gegensatz zur Ausführungsform in Fig. 4 auch für Lichtstrahlen erfolgen, bei denen der Auftreffwinkel $\theta < \theta_c$ ist. Die Beschichtung 10 kann als ein- oder mehrlagige Beschichtung ausgebildet sein und beispielsweise Silberschichten oder Aluminiumschichten umfassen. Bei dem in Fig. 5 gezeigten Ausführungsbeispiel ist die auskoppelnde Seitenfläche 3 die Seitenfläche mit der größeren Rauheit. Es ist jedoch auch möglich, die Seitenfläche 3 als glatte Oberfläche und die gegenüberliegende Seitenfläche 2 als raue Seitenfläche auszubilden. Das Substrat 100 kann amorph oder kristallin sein. Gemäß einer Ausführungsform wird das Substrat 100 durch Quarzglas, Saphir oder kristallines $CaF_2$ oder kristallines $MgF_2$ gebildet. Als besonders vorteilhaft hat sich die Verwendung von wasserangereichtem Quarzglas als Material für das Substrat 100 herausgestellt. So weist wasserangereichter Quarz bzw. Quarzglas eine gegenüber konventionellem Quarz verringerte Dämpfungsneigung auf. Auch die Polarisationsneigung des wasserangereichten Quarzglases ist deutlich geringer.

Fig. 6 zeigt eine weitere Ausführungsform des Lichtverteilungselements 1. Hier weist das Substrat 100 auf beiden Seitenflächen 2, 3 Beschichtungen auf. Auf der Seitenfläche 2 befindet sich analog zu dem in Fig. 5 gezeigten Ausführungsbeispiel eine reflektierende Beschichtung 10. Die auskoppelnde Seitenfläche 3 ist ebenso wie in dem in Fig. 5 gezeigten Beispiel strukturiert, weist darüber hinaus jedoch eine teilreflektierende Beschichtung 11 auf. Durch die teilreflektierende Beschichtung 11 kann der Anteil des ausgekoppelten Lichts beeinflusst werden.

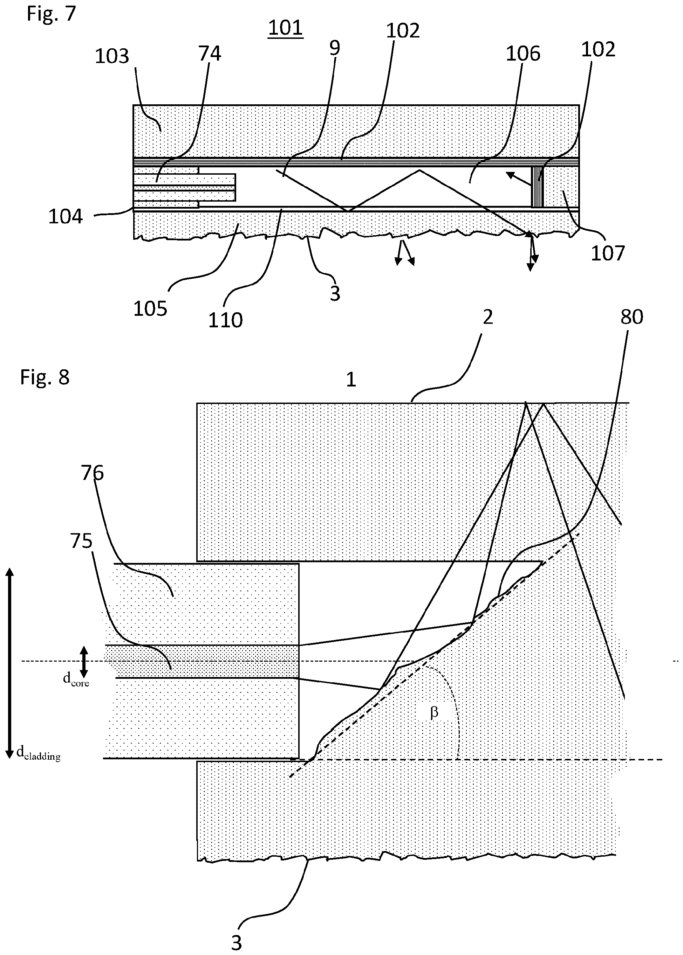

Fig. 7 zeigt einen schematischen Querschnitt durch ein Lichtverteilungselement 101. Das Lichtverteilungselement 101 umfasst mehrere Substratkomponenten 103, 104, 105 und 107. Komponente 105 sowie vorzugsweise die Komponenten 103, 104 und 107 bestehen aus einem für das eingekoppelte Licht transparenten Material. Die auskoppelnde Seitenfläche 3 wird durch die Substratkomponente 105 gebildet. Die Substratkomponenten 103, 104, 105 und 107 sind so angeordnet, dass sich zwischen ihnen ein mit Luft oder einem Gas gefüllter oder evtl. sogar evakuierter Hohlraum 106 bildet. Durch eine Öffnung in der Komponente 104 wird ein Lichtleiter 74 in den Hohlraum 106 geführt, wo das Licht 9 abgegeben wird. Der Hohlraum 106 wird durch Oberflächen der Komponenten 104, 105 und 107 begrenzt. Die Komponente 107 befindet sich gegenüber von der Komponenten 104, die Komponenten 103 und 105 oberhalb der Komponente 104. Die Komponenten 104 und 107 können auch als eine Komponente, d.h. einstückig ausgebildet sein. Bei dieser Ausführungsform umschließt diese Komponente den Hohlraum 106. Die Oberflächen der Komponenten 103 und 107, welche den Hohlraum 106 begrenzen, sind mit reflektierenden Beschichtungen 102 versehen. Die Oberfläche der Komponente 105, welche den Hohlraum 106 begrenzt, weist eine teilreflektierende Beschichtung 110 auf. Das Licht tritt aus dem Lichtleiter 74 in den Hohlraum106 aus und breitet sich aus. Ein beispielhafter Strahlengang ist durch die Linie 9 dargestellt. Trifft das Licht auf die reflektierenden Beschichtungen 102, so wird es zurück in den Hohlraum 106 gelenkt. Die auf der Komponente 105 abgeschiedene Beschichtung 110 ist eine teilreflektierende Beschichtung, somit wird ein Teil des Lichts in die Komponente 105 durch die Beschichtung 110 eingekoppelt. Der eingekoppelte Teil des Lichts breitet sich innerhalb der Komponente 105 aus und wird an deren strukturierten Seitenfläche 3 ausgekoppelt und gestreut. Über die Beschichtung 110 bzw. deren Transmissionsgrad kann der Anteil des Lichts eingestellt werden, welcher durch die Komponente 105 ausgekoppelt wird. Durch wiederholte Reflexion innerhalb des Hohlraums 106 kann dabei über die gesamte Auskoppelfläche 3 eine homogene Lichtintensität erhalten werden.

Die in Fig. 7 gezeigte Ausführungsform eignet sich insbesondere für Anwendungen, bei denen das Licht seitlich in das Lichtverteilungselement 1, 101 eingekoppelt werden soll. Durch den komplexeren Aufbau und Auswahl der Beschichtungen der Komponenten 103, 104, 105 und 107 können besonders homogen verteilte Lichtintensitäten erhalten werden. Ebenso ist denkbar, dass eine der Komponenten 103 und/oder 105, anders als hier dargestellt, eine andere als eine planare Geometrie annehmen, also bspw. als Segment einer Kugel, wie z.B. ein sogenanntes Uhrglas, oder generell mit einer 3d-geformten Oberfläche ausgebildet werden. Damit kann ebenfalls in die Lichtverteilung oder Homogenisierung der Lichtintensitäten eingegriffen werden. Weiterhin können die Lichtverteilungselemente 101 nach den bisher gezeigten Figuren, an der der Behandlungsseite abgewandten Seite, auch von weiteren, auch für UV-Licht nicht transparenten, Komponenten überdeckt und/oder umschlossen werden. Dies bspw., um eine Abstrahlung von UV-Licht nach außen von der Behandlungsseite weg zu unterbinden.

Fig. 8 zeigt einen schematischen Querschnitt eines Lichtverteilungselements 1 mit schräger Einkoppelfläche 80. Die Einkoppelfläche 80 weist einen Winkel β zur Waagerechten im Bereich von 10 ° bis 80°, bevorzugt im Bereich von 30° bis 50° auf. Der Lichtleiter 75 ist am distalen Ende angeschrägt oder aufgespleißt, so dass der aus dem Lichtleiter 75 austretende Lichtstrahl bereits aufgeweitet wird. Durch Reflexion des Lichtes an der schrägen Einkoppelfläche 80 wird das Licht zusätzlich im Lichtverteilungselement 1 homogen verteilt, bevor es durch die auskoppelnde Seitenfläche 3 austritt und an der rauen bzw. strukturierten Oberfläche gestreut wird. Die homogenisierende Wirkung des Lichtverteilungselements 1 kann weiter verbessert werden, indem auch die Oberfläche der Einkoppelfläche 80 strukturiert oder angeraut ist und so den ursprünglichen Lichtkegel, der von der Faser ausgeht, weiter durch Streuung oder Beugung verbreitert.

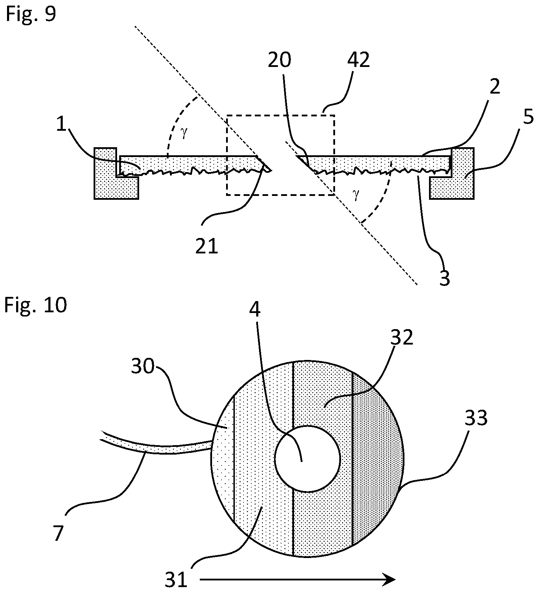

In Fig. 9 ist ein weiteres Ausführungsbeispiel des Lichtverteilungselements 1 dargestellt. In dieser Ausführungsform verläuft die Durchführungsöffnung 42 nicht senkrecht, sondern schräg durch das Lichtverteilungselement 1. Somit weist die Durchführungsöffnung 42 angeschrägte Wandungen 20, 21 auf. Die Wandungen bilden mit den Seitenflächen 2, 3 des Lichtverteilungselements 1 einen Winkel γ, der vorzugsweise im Bereich von 5 bis 50° liegt. Durch die angeschrägte Durchgangsöffnung 42 eignet sich die Beleuchtungseinrichtung besonders gut für die Verwendung mit Kathetern, die in einem flachen Winkel gelegt werden, da die Katheter durch das Loch in einem definierten Winkel zur Hautoberfläche geführt werden. In dem in Fig. 9 dargestellten Ausführungsbeispiel ist die Durchgangsöffnung 42 mittig innerhalb des Lichtverteilungselement 1 angeordnet. Alternativ ist es jedoch auch möglich, die Durchgangsöffnung 42 abhängig vom Winkel γ außermittig zu platzieren.

Fig. 10 stellt eine schematische Aufsicht auf die auskoppelnde Seitenfläche einer weiteren Ausführung eines Lichtverteilungselements dar. Die auskoppelnde Seitenfläche ist beispielhaft in die vier Teilbereiche 30, 31, 32 und 33 unterteilt. Das Licht wird über den Lichtleiter 7 an der Stirnseite des Lichtverteilungselements (nicht dargestellt) im Bereich des Teilbereichs 30 eingekoppelt. Die Teilbereiche 30, 31, 32, 33 unterscheiden sich hinsichtlich ihrer Rauheit RMS, wobei die Rauheit vom Teilbereich 30 bis zum Teilbereich 33 zunimmt. Dieser Verlauf der Rauheit wird durch den Pfeil symbolisiert. Die Rauheit nimmt mit steigendem Abstand von der Einkoppelstelle zu. Hierdurch ist der Anteil des Lichts, welches durch die auskoppelnde Seitenfläche das Lichtverteilungselement Auskopplung in Bereichen, die nahe an der Einkoppelstelle liegen, geringer als in Bereichen, die weiter entfernt von der Einkoppelstelle sind. Durch die Zunahme der Rauheit und die damit verbundene Vergrößerung des Auskopplungsgrades mit zunehmenden Abstand zur Einkoppelstelle wird somit ein Verlust der Lichtintensität innerhalb des Lichtverteilungselement 1 kompensiert. Dies gewährleistet eine homogene Lichtintensität des ausgekoppelten Lichtes über die gesamte auskoppelnde Seitenfläche 3 Die lokale Änderung der Rauheit der Seitenfläche 2 kann vorteilhaft in mehr als vier Teilbereichen und besonders bevorzugt für eine homogene Lichtverteilung graduell erfolgen. So erhöht sich gemäß einer anderen Ausführungsform die Rauheit vom Teilbereich 30 hin zum Teilbereich 33 kontinuierlich. Je nach Ausführung mit einem oder auch mehreren Lichtleitern 7 zur Einkopplung von Licht, können auch andere als die hier gezeigte lineare, ggf. graduelle, Rauheitsänderung vorteilhaft oder notwendig sein. So kann die Rauheitsänderung auch zirkulär ausgebildet oder konzentrisch zur oder um die Durchführungsöffnung 4 ausgelegt werden.

Vorteilhaft soll also die Rauheit mit steigendem Abstand von der Einkoppelstelle zunehmen und die Auskopplung von Licht aus dem Lichtverteilungselement mit zunehmenden Abstand von der Einkoppelstelle begünstig beziehungsweise variiert werden. Diese Zunahme oder Variation kann in Stufen bzw. diskreten aufeinanderfolgenden Bereichen mit je konstanter Rauheit, wie auch als Gradient, also kontinuierlicher Veränderung der Rauheit, als Rauheitsprofil, ausgelegt sein. Sowohl die Stufen als auch ein etwaiger Gradient können linear ausgebildet sein, d.h. die Rauheit nimmt von der Einkoppelstelle linear zu. Ebenso kann die Veränderung, insbesondere die Zunahme der Rauheit auch beispielsweise einer e-Funktion folgend, also exponentiell verlaufend oder variierend, oder einer anderen Funktion folgend ausgelegt werden, um optimale oder auch spezielle Ausleuchtungen zu erreichen. Insbesondere im Falle der Einkopplung von Licht an mehreren Stellen des Lichtverteilungselementes 1 über zwei oder mehr Lichtleiter 7 ergibt sich, quasi durch die Überlagerung der einzelnen Rauheitsprofile ausgehend von je einer Einkoppelstelle, ein Überlagerungs-Rauheitsprofil. Bei einem kreisförmigen Lichtverteilungselement 1 mit mehreren radial gleichmäßig angeordneten Einkoppelstellen, wird sich dieses Überlagerungs-Rauheitsprofil mit zunehmender Anzahl Einkoppelstellen einem zirkulären oder konzentrischen Verlauf annähern. Entsprechend der Geometrie des Lichtverteilungselements 1, der Anzahl der Einkoppelstellen und der geforderten Ausleuchtung können sich komplexe gestufte oder gradierte Rauheits- beziehungsweise Überlagerungs-Rauheitsprofile ergeben beziehungsweise eingestellt werden.

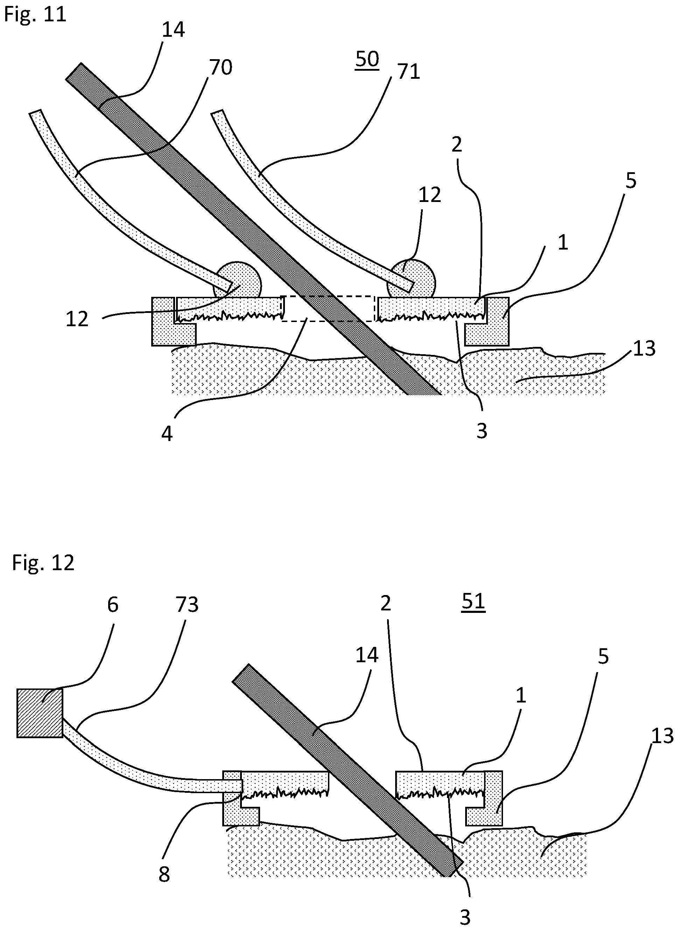

Fig. 11 zeigt eine Vorrichtung zur Sterilisation der Haut 50 gemäß einem Ausführungsbeispiel im Querschnitt. Die Vorrichtung 50 umfasst eine Beleuchtungseinrichtung mit einem Lichtverteilungselement 1, einer Halterung 5, Lichtleitern 70, 71, einer Lichtquelle (nicht dargestellt) sowie einen Katheter 14. Das Lichtverteilungselement 1 ist auf einer Haltevorrichtung 5 montiert, wobei die Haltevorrichtung 5 ein L-förmiges Profil aufweist und so über das Lichtverteilungselement 1 hinausgeht, dass sie als Abstandhalter zwischen dem Lichtverteilungselement 1 und der Haut 13 fungiert. Hierbei hat die Haltevorrichtung 5 zumindest punktuell Hautkontakt. In dieser Ausführungsform ist die Haltevorrichtung aus einem organischen Polymer, vorzugsweise aus einem Polysiloxan oder einem Polycarbonat gefertigt. Die Lichteinkopplung erfolgt über die Seitenfläche 2 des Lichtverteilungselements 1, welche der auskoppelnden Seitenfläche 3 gegenüberliegt. Die Lichtleiter 70, 71 sind durch ein Glaslot oder einem Kleber 12 mit dem Lichtverteilungselement 1 stoffschlüssig verbunden. Die Durchführungsöffnung 4 ist in der Mitte des Lichtverteilungselements 1 angeordnet. Durch die Durchführungsöffnung 4 wird ein Katheter 14 geführt. Das Lichtverteilungselement 1 ist dabei so ausgerichtet, dass die auskoppelnde Seitenfläche 3 zur Hautoberfläche 13 gerichtet ist. Somit wird der Bereich der Hautoberfläche 13, welcher unterhalb des Lichtverteilungselements 1 liegt, mit dem ausgekoppelten Licht bestrahlt und so desinfiziert bzw. sterilisiert. Durch die Streuung des auskoppelnden Lichtes an der rauen Seitenfläche 3 wird dabei ein Beleuchtungsfeld mit homogener oder zumindest weitgehend homogener Lichtintensität erzeugt.

Fig. 12 zeigt ein weiteres Ausführungsbeispiel einer Vorrichtung zur Sterilisation der Haut 51. Der Aufbau der Vorrichtung 51 entspricht in weiten Teilen dem Aufbau des in Fig. 11 dargestellten Ausführungsbeispiels 50. Abweichend hiervon erfolgt die Einkopplung des Lichtes durch den Lichtleiter 73 jedoch über die Stirnseite 8 bzw. einen Teilbereich der umlaufenden Kante, welche die Stirnseite 8 bildet.

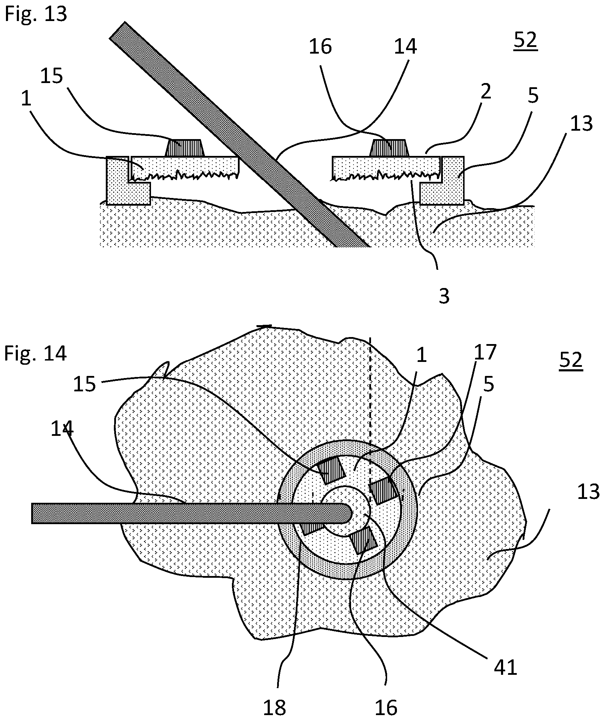

Ein weiteres Ausführungsbeispiel einer Vorrichtung zur Sterilisation der Haut 52 wird in den Fig. 13 schematisch dargestellt, wobei Fig. 13 einen schematischen Querschnitt des Ausführungsbeispiels und Fig. 14 eine schematische Darstellung in Aufsicht zeigt. Im Unterschied zu den in den Fig. 11 und 12 gezeigten Ausführungsbeispielen 50, 51 sind bei diesem Ausführungsbeispiel die Lichtquellen 15, 16, 17, 18 auf der Seitenfläche 2 des Lichtverteilungselements 1 angeordnet. Vorzugsweise handelt es sich bei den Lichtquellen 15, 16, 17, 18 um UV-LEDs, welche UV-Licht im Wellenlängenbereich von 250 bis 300 nm emittieren. Die Lichtquellen 15, 16, 17, 18 können mit der Seitenfläche 2 des Lichtverteilungselements 1 verklebt sein oder auch durch eine Vorrichtung gehalten werden (nicht dargestellt). Das von den Lichtquellen 15, 16, 17, 18 emittierte Licht wird somit direkt in das Lichtverteilungselement 1 eingekoppelt. Im gezeigten Ausführungsbeispiel 52 weist die Vorrichtung vier Lichtquellen 15, 16, 17, 18 auf, welche jeweils im gleichen Abstand zum Mittelpunkt des Lichtverteilungselements 1 angeordnet sind und untereinander äquidistante Abstände aufweisen. Durch die Verwendung mehrerer Lichtquellen 15, 16, 17, 18 erfolgt bereits die Einkopplung des Lichtes gleichmäßig verteilt über das Lichtverteilungselement 1, was sich auch auf die Homogenität der ausgekoppelten Lichtintensität auswirkt.

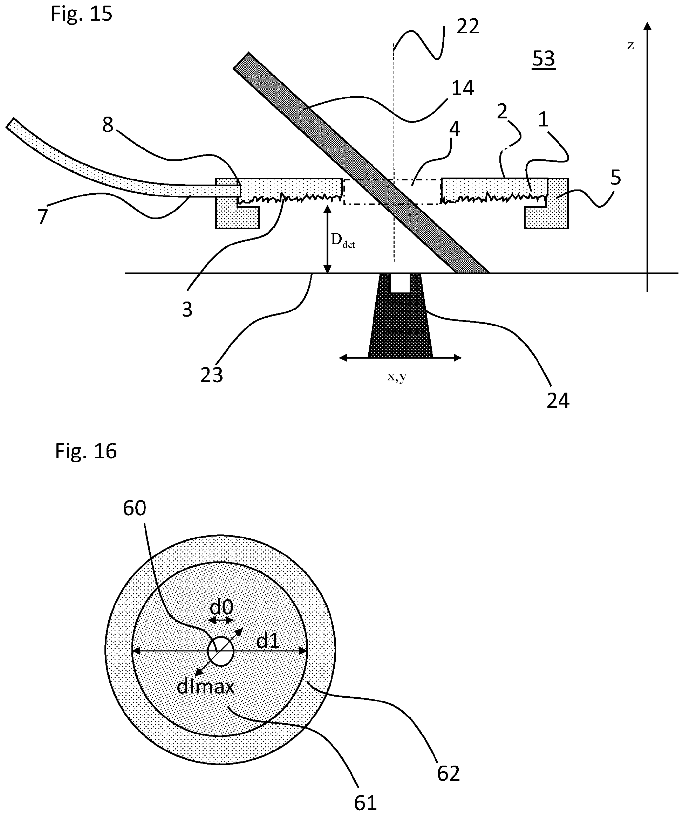

Fig. 15 zeigt eine schematische Darstellung eines Messverfahrens zur Bestimmung der Homogenität des vom Lichtverteilungselement abgegebenen Lichts. Unter die Vorrichtung zur Sterilisation der Haut 53 wird ein Detektor 24 entlang der Detektionsebene 23 und ausgehend vom Mittelpunkt 22 der Durchführungsöffnung 4 bewegt. Hierbei wird kontinuierlich die Lichtintensität gemessen, so dass eine ortsaufgelöste Lichtintensität, d.h. die Lichtintensität in Abhängigkeit vom Abstand vom Mittelpunkt 22 erhalten wird. Der Detektor 24 umfasst eine Photodiode 24 als Sensor. Als Photodiode kann eine Si-Photodiode, beispielsweise eine Photodiode des Typs Thorlabs FDS010 versendet werden. Der Detektor ist für einen Wellenlängenbereich zwischen 200 nm und 290 nm kalibriert und kann Energien unterhalb von 1 mW mit einer Auflösung von zumindest $50\,\mu$W detektieren. Zur Leistungskalibrierung der Photodiode können beispielsweise geeichte Detektoren des Typs MKS Ophir, PD300R-UV oder Coherent Laser PowerMax-USB PS10 verwendet werden.

Der Detektor 24 entlang der Detektionsebene 23 in x- und y-Richtung über zumindest einen Bereich von 20 mm bewegt und die Lichtenergie wird mit einer räumlichen Auflösung von zumindest 50 $\mu$m gemessen. Die Detektionsebene 23 weist dabei einen Abstand zur auskoppelnden Seitenfläche des Lichtverteilungselements von $D_{det}$=10 mm auf. Zur Ermittlung der Lichtintensität werden die gemessenen Lichtenergien durch die Detektionsfläche der Photodiode geteilt.

Fig. 16 zeigt schematisch eine Aufsicht auf eine von einer erfindungsgemäßen Vorrichtung zur Sterilisation der Haut beleuchteten Fläche 62. Die Fläche 60 entspricht der Fläche der Einstichstelle des Katheters und weist einen Durchmesser $d_0$ auf. Der Durchmesser $d_0$ entspricht hierbei dem Durchmesser des Katheters. Die Fläche 61 entspricht einer mit der in Fig. 15 dargestellten Vorrichtung vermessenen Bereich und weist einen Durchmesser $d_1$ auf.

Ein Ausführungsbeispiel der Vorrichtung sieht dabei vor, dass die Bestrahlungsintensität des vom Lichtverteilungselement ausgekoppelten UV-Lichts auf einer Fläche 62, die unterhalb des Lichtverteilungselementes angeordnet ist, so homogenisiert ist, dass entlang einer kreisförmigen Begrenzungslinie $d_1$ in einem wählbaren Abstand vom Mittelpunkt der Öffnung bis maximal 2 cm von diesem Mittelpunkt das Verhältnis des Maximums der Bestrahlungsintensität und des Minimums der Bestrahlungsintensität ein Verhältnis von höchstens 3, vorzugsweise von höchstens 2 beträgt. Es gilt somit:

$$I_{max,d1}/I_{min,d1} \leq 3, \text{ bevorzugt} \leq 2 \text{ und besonders bevorzugt} \leq 1$$

Mit $d_1$ < 4 cm.

Zudem weist die bestrahlte Fläche 62 ihr Intensitätsmaximum bei einem Abstand $d_{Imax}$ auf, wobei für $d_{Imax}$ gilt: $d_{Imax} \leq 3$ cm, bevorzugt $\leq 2$ cm. Der Bereich 63 mit der maximalen Lichtintensität ist somit höchstens 1,5 cm, bevorzugt höchstens 2 cm vom Mittelpunkt der Einstichstelle mit dem Durchmesser $d_0$ entfernt.

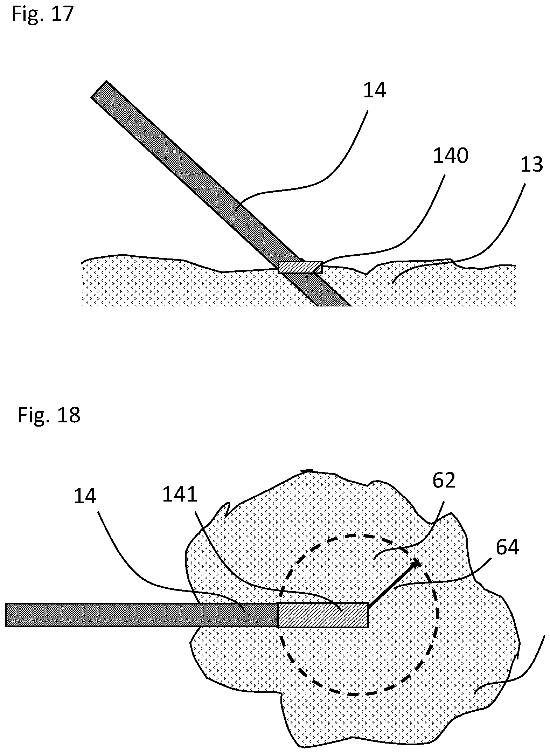

In den Fig. 17 und 18 sind schematisch die Bereiche einer minimalen Abschattung 140 und einer maximalen Abschattung 141 innerhalb der beleuchteten Fläche 62 durch den Katheter 14 dargestellt. Fig. 17 ist eine schematische Aufsicht, bei Fig. 18 handelt es sich um eine schematische Seitenansicht. Der Bereich 140, der unabhängig von der Anordnung der Beleuchtungsvorrichtung stets durch den Katheter abgeschattet wird, ist die Einstichstelle des Katheters. Die Fläche des Bereichs 140 entspricht somit der Fläche der Einstichstelle bzw. der Querschnittsfläche des Katheters und ergibt sich wie folgt:

$$A_{in} = \pi * d_c{}^2/(2*\cos\alpha)$$

mit $d_c$= Katheterdurchmesser und $\alpha$ dem Einstichwinkel des Katheters. Somit weist die Vorrichtung zwangsläufig einen Anteil an beschatteter Fläche $S_{min}$ auf, die wie folgt berechnet werden kann:

$$S_{min} = A_{in}/A = d_c{}^2/(4R^2*\cos\alpha)$$

Mit A= beleuchtete Fläche, R= Radius der beleuchteten Fläche.

Bei einer Vorrichtung mit einer beleuchteten Fläche mit einem Durchmesser von 15 mm und einem Katheterdurchmesser von 2,1 mm ergibt sich somit ein minimaler Abschattungsanteil $S_{min}$=2%.

Der maximale Bereich $A_c$ der durch den Katheter beschattet werden kann, ist mit dem Bezugszeichen 141 gekennzeichnet und kann wie folgt berechnet werden:

$$A_c = R*d_c$$

Mit R= Radius der beleuchteten Fläche und $d_c$= Katheterdurchmesser.

Entsprechend gilt für den maximalen Abschattungsanteil $S_{max}$

$$S_{max} = A_c/A = d_c/(\pi * R)$$

Bei dem oben genannten Abmessungen dc=2,1 mm und R = 7,5 mm ergibt sich somit ein theoretischer maximaler abgeschatteter Anteil $S_{max}$ von 9%.

Durch den Einsatz des Lichtverteilungselementes kann bei den erfindungsgemäßen Vorrichtungen dieser Anteil jedoch deutlich reduziert werden. Gemäß einer Ausführungsform gilt daher für den Anteil der abgeschatteten Bereichen $S_{real}$:

$S_{min} < S_{real} < 0,7 * S_{max}$, bevorzugt $S_{min} < S_{real} \leq 0,5*S_{max}$ und besonders bevorzugt $S_{min} < S_{real} \leq 0,3* S_{max}$.

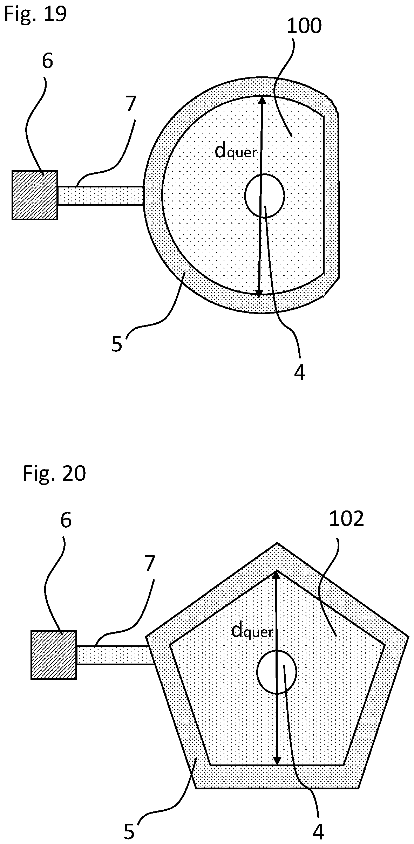

In den Fig. 19 und 20 sind schematisch weitere Ausführungsformen der Beleuchtungsvorrichtung in Aufsicht dargestellt. Fig. 19 zeigt hierbei eine Beleuchtungsvorrichtung, bei der das Lichtverteilerelement 100 keinen vollständigen Kreis bildet, sondern D-förmig ist. Der Pfeil zeigt die maximale Querabmessung $d_{quer}$. Das in Fig. 20 dargestellte Lichtverteilerelement 102 weist dagegen ein fünfeckiges, d.h. polygonales Lichtverteilerelement auf.

Bezugszeichenliste

| 1, 100, 101, 102 | Lichtverteilungselement |
|---|---|
| 2 | Seitenfläche des Lichtverteilungselement s ohne Lichtauskopplung |
| 3 | Auskoppelnde Seitenfläche des Lichtverteilungselements |
| 4, 40, 41 | Durchführungsöffnung |
| 5 | Halterungselement |
| 6 | Lichtquelle |
| 7, 70, 71, 73,74, 75 | Lichtleiter |
| 8 | Stirnseite des Lichtverteilungselements 1 |
| 9 | Lichtstrahl |
| 10, 102 | Reflektierende Beschichtung |
| 11, 110 | Teilreflektierende Beschichtung |
| 12 | Glaslot |
| 13 | Haut |
| 14 | Katheter |
| 15, 16, 17, 18 | UV-LED |
| 20, 21 | Wandungen der Durchführungsöffnung |
| 22 | Mittelpunkt des Lichtverteilungselements 1 |
| 23 | Detektionsebene |
| 24 | Bewegliche Photodiode |
| 30, 31, 32, 33 | Teilbereiche der Seitenfläche 3 |
| 50, 51, 52, 53 | Vorrichtung zur Sterilisation der Haut |
| 60 | Einstichpunkt |
| 61 | Beleuchtete Fläche |
| 62 | Bereich von 61, in der die Lichtintensität ermittelt wird |
| 63 | Bereich von 61 mit der maximalen Lichtintensität |
| 64 | Detektor |
| 76 | Ummantelung des Lichtleiters 75 |

(fortgesetzt)

| 103, 104, 105, 1075 und 107 | Komponenten von 101 |
|---|---|
| 106 | Luft |
| 140 | Minimaler Abschattungsbereich |
| 141 | Maximaler Abschattungsbereich |
| | |
| | |

## Patentansprüche

1. Beleuchtungsvorrichtung umfassend zumindest eine Lichtquelle (6), welche Licht einer Wellenlänge im Bereich von 180 nm bis 360 nm emittiert, und ein Lichtverteilungselement (1) mit zwei gegenüberliegenden Seitenflächen (2, 3), wobei das Lichtverteilungselement (1) ein für das eingekoppelte Licht transparentes oder zumindest weitgehend transparentes Material umfasst, wobei das Licht der Lichtquelle (6) in das Lichtverteilungselement (1) eingekoppelt wird und aus zumindest einer der beiden Seitenflächen (2, 3) des Lichtverteilungselements (1) austritt, wobei das Lichtverteilungselement (1) Strukturen zur Streuung des eingekoppelten Lichts aufweist, um das Licht zumindest teilweise so abzulenken, dass es aus zumindest einer der Seitenflächen (2, 3) austritt, wobei das Lichtverteilungselement (1) zumindest eine Durchgangsöffnung (4, 40, 41) aufweist, die sich von der einen Seitenfläche (2) des Lichtverteilungselements (1) zur anderen Seitenfläche (3) erstreckt.

2. Beleuchtungsvorrichtung gemäß dem vorstehenden Anspruch, wobei die Beleuchtungsvorrichtung zur Verwendung in einem medizintechnischen Behandlungssystem geeignet ist und bevorzugt die Durchgangsöffnung als Durchführung für einen Katheter (14) ausgebildet ist.

3. Beleuchtungsvorrichtung gemäß einem der vorstehenden Ansprüche, wobei ein Bereich (62), der von dem aus der auskoppelnden Seitenfläche (3) des Lichtverteilungselements (1) austretenden Lichts bestrahlt wird, eine Fläche im Bereich von 1 cm$^2$ bis 25 cm$^2$, bevorzugt im Bereich von 1 bis 20 cm$^2$, besonders bevorzugt im Bereich von 1 bis 8 cm$^2$ und ganz besonders bevorzugt im Bereich von 1 bis 4 cm$^2$ aufweist.

4. Beleuchtungsvorrichtung gemäß einem der vorstehenden Ansprüche, wobei das Lichtverteilungselement (1) kreisförmig, ellipsoid oder D-förmig geformt ist und bevorzugt eine maximale Querabmessung im Bereich von 1 bis 8 cm, besonders bevorzugt im Bereich von 2 bis 6 cm aufweist.

5. Beleuchtungsvorrichtung gemäß einem der vorstehenden Ansprüche, wobei die Lichtquelle (6) UV-Licht einer Wellenlänge im Bereich von 180 bis 250nm, bevorzugt in einem Bereich von 200 bis 230 nm oder in einem Bereich von 230 bis 300 nm, bevorzugt in einem Bereich von 250 bis 270 nm emittiert.

6. Beleuchtungsvorrichtung gemäß einem der vorstehenden Ansprüche, wobei das Lichtverteilungselement (1) ein Material umfasst, dessen Dämpfung im Wellenlängenbereich des eingekoppelten Lichts weniger als -3 dB/cm vorzugsweise weniger als -1 dB/cm beträgt und/oder das Lichtverteilungselement (1) amorphe Materialien, vorzugs-weise UV-transparentes $SiO_2$ oder ein kristallines Material, vorzugsweise Saphir, $MgF_2$ oder $CaF_2$, besonders bevorzugt wasserangereichtertes Quarzglas, umfasst.

7. Beleuchtungsvorrichtung gemäß einem der vorstehenden Ansprüche, wobei zumindest eine Seitenfläche (2, 3) des Lichtverteilungselements (1), bevorzugt die auskoppelnde Seitenfläche (3), zumindest in Teilbereichen eine Rauheit RMS im Bereich von 1 bis 400 nm, bevorzugt im Bereich von 10 bis 200 nm und besonders bevorzugt im Bereich von 50 bis 150 nm aufweist.

8. Beleuchtungsvorrichtung gemäß dem vorstehenden Anspruch, **gekennzeichnet durch** zumindest eines der Merkmale

   - die auskoppelnde Seitenfläche (3) des Lichtverteilungselements (1) weist lokal unterschiedliche Rauheiten auf, wobei die Rauheiten vorzugsweise einen Gradientenverlauf aufweisen, vorzugsweise im Bereich von 0,2 nm bis 150 nm, bevorzugt im Bereich von 0,2 nm bis 100 nm, besonders bevorzugt im Bereich 0,3 nm bis 80 nm,

- zumindest eine Seitenfläche (2, 3) des Lichtverteilungselements (1) weist Mikrostrukturierung zur Einstellung der lokalen Intensität des ausgekoppelten Lichts auf,

- eine der beiden Seitenflächen (2, 3) des Lichtverteilungselements (1) weist eine höhere Rauheit als die gegenüberliegende Seitenfläche (2, 3) auf, bevorzugt weist die auskoppelnde Seitenfläche (3) eine höhere Rauheit auf und/oder eine der Seitenflächen (2, 3) weist eine Rauheit RMS von weniger als 0,5 nm, bevorzugt weniger als 0,2 nm und besonders bevorzugt weniger als 0,1 nm auf,

- zumindest eine der Seitenflächen (2, 3) des Lichtverteilungselements (1) weist eine reflektierende Beschichtung, bevorzugt eine reflektierende Aluminium- oder Silberbeschichtung, auf,

- das Lichtverteilungselement (1) ist als kreisförmige Scheibe mit einem Durchmesser im Bereich von 20 bis 30 mm und einer Dicke im Bereich von 0,4 bis 1,5 mm ausgebildet, wobei Durchführungsöffnung (4, 40, 41) in der Mitte der Scheibe angeordnet ist und die Durchführungsöffnung (4, 40, 41) einen Durchmesser im Bereich von 2 bis 6 mm aufweist,

- das Lichtverteilungselement (1) ist in einem umlaufenden Halterungselement (5) fixiert, wobei ein Bereich des Halterungselements (5) über auskoppelnde Seitenfläche (3) des Lichtverteilungselements (1) hinausragt.

9. Beleuchtungsvorrichtung gemäß dem vorstehenden Anspruch, wobei das Halterungselement (5) ein organisches Polymer, vorzugsweise ein Polysiloxan oder ein Polycarbonat umfasst.

10. Beleuchtungsvorrichtung gemäß einem der vorstehenden Ansprüche, wobei das Licht der Lichtquelle (6) über zumindest einen Lichtleiter (7, 74) in das Lichtverteilungselement (1) eingekoppelt wird.

11. Beleuchtungsvorrichtung gemäß dem vorstehenden Anspruch, **gekennzeichnet durch** zumindest eines der Merkmale

- der Lichtleiter (7, 74) ist mit dem Lichtverteilungselement (1) verspleißt oder verklebt,
- der Lichtleiter (7, 74) am distalen Ende, welches mit dem Lichtverteilungselement (1) verbunden ist, eine Numerische Apertur NA > 0,1, bevorzugt größer 0,2 aufweist.
- der Lichtleiter (7, 74) ist über die Seitenfläche (2) des Lichtverteilungselements (1), welche der auskoppelnden Seitenfläche (3) gegenüber liegt, mit dem Lichtverteilungselement (1) verbunden,
- der Lichtleiter (7, 74) ist über eine Stirnseite (8) des Lichtverteilungselements (1) mit dem Lichtverteilungselement (1) verbunden.

12. Beleuchtungsvorrichtung gemäß dem vorstehenden Anspruch, wobei die Einkopplung des Lichtes über eine schräge Fläche (80) des Lichtverteilungselements (1) erfolgt und bevorzugt die schräge Fläche einen Winkel $\alpha$ zu den Seitenflächen (2, 3) des Lichtverteilungselements (1) im Bereich von 30 bis 50° aufweist.

13. Beleuchtungsvorrichtung gemäß dem vorstehenden Anspruch, wobei die schräge Einkoppelfläche (80) eine Rauheit RMS von > 1nm, bevorzugt >2 nm, besonders bevorzugt > 10 nm und ganz besonders bevorzugt > 50 nm aufweist.

14. Beleuchtungsvorrichtung gemäß einem der vorstehenden Ansprüche 1 bis 14, wobei das Licht über die Seitenfläche (2) des Lichtverteilungselements (1), welche der auskoppelnden Seitenfläche (3) gegenüber liegt, eingekoppelt wird und die Lichtquelle (6) mit dieser Seitenfläche (2) formschlüssig oder stoffschlüssig verbunden ist.

15. Beleuchtungsvorrichtung gemäß dem vorstehenden Anspruch, wobei die Beleuchtungsvorrichtung zumindest eine, bevorzugt mehrere Lichtquellen (6) aufweist und die Lichtquellen (6) vorzugsweise UV-LEDs (15, 16, 17, 18) sind.

16. Vorrichtung (50, 51, 52, 53) zur Sterilisation der Haut (13) umfassend eine Beleuchtungseinrichtung gemäß einem der vorstehenden Ansprüche sowie einen Katheter (14), wobei der Katheter (14) so durch die Durchgangsöffnung (4, 40) geführt ist, dass das Ende des Katheters (14), mit welchem der Katheter (14) durch die Haut (13) in den Patienten eingeführt wird, sich auf der Seite des Lichtverteilungselements (1) mit der auskoppelnden Seitenfläche (3) befindet und wobei die Beleuchtungseinrichtung ein umlaufendes Halterungselement (5) umfasst, das zumindest auf der auskoppelnden Seitenfläche (3) des Lichtverteilungselements (1) über das Lichtverteilungselement (1) herausragt und einen Abstandhalter zwischen der Haut (13) und dem Lichtverteilungselement (1) bildet.

Fig. 1

Fig. 2

Fig. 3

Fig. 4

Fig. 5

Fig. 6

Fig. 7

103   74   **101**   9   102   106   102

104

105   110   3   107

Fig. 8

1   2   80

76

75

$d_{core}$

$d_{cladding}$

β

3

Fig. 9

20   42

1   γ

2   5

21

3

Fig. 10

4   32

30   33

7

31 →

Fig. 11

Fig. 12

Fig. 13

15

1

16    14    52

2    5    13

3

Fig. 14

15

14

1    17    52

5

13

18    16    41

Fig. 15

$D_{dct}$

x,y

z

53

Fig. 16

d0

d1

d1max

Fig. 17

Fig. 18

Fig. 19

6

7

100

$d_{quer}$

5

4

Fig. 20

6

7

102

$d_{quer}$

5

4

Europäisches
Patentamt

European
Patent Office

Office européen
des brevets

**EUROPÄISCHER RECHERCHENBERICHT**

Nummer der Anmeldung

EP 25 15 3309

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | WO 2022/248691 A1 (ASEPTUVA GMBH [CH]) 1. Dezember 2022 (2022-12-01) * Seite 28, Zeile 14 - Seite 39, Zeile 3 * * Abbildungen 1,2,4,6 * ----- | 1-16 | INV. A61N5/06 |
| A | US 2003/018373 A1 (ECKHARDT RICHARD [US] ET AL) 23. Januar 2003 (2003-01-23) * Absätze [0008], [0066] - [0072] * * Abbildungen 6,7 * ----- | 1-16 | |
| A | US 2022/323787 A1 (ELTORAI ADAM E M [US] ET AL) 13. Oktober 2022 (2022-10-13) * Absatz [0025] * * Abbildung 1 * ----- | 1-16 | |
| A | US 2015/174426 A1 (ST GERMAIN MARK DAVID [US] ET AL) 25. Juni 2015 (2015-06-25) * Absätze [0024], [0029] * * Abbildungen 1,2 * ----- | 1-16 | |

**RECHERCHIERTE SACHGEBIETE (IPC)**

A61N

Der vorliegende Recherchenbericht wurde für alle Patentansprüche erstellt

| Recherchenort | Abschlußdatum der Recherche | Prüfer |
|---|---|---|
| München | 12. Juni 2025 | Kretschmann, Jana |

EPO FORM 1503 03.82 (P04C03)

**ANHANG ZUM EUROPÄISCHEN RECHERCHENBERICHT**
**ÜBER DIE EUROPÄISCHE PATENTANMELDUNG NR.**                    EP 25 15 3309

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

12-06-2025

| Im Recherchenbericht angeführtes Patentdokument | Datum der Veröffentlichung | Mitglied(er) der Patentfamilie | | Datum der Veröffentlichung |
|---|---|---|---|---|
| WO 2022248691 A1 | 01-12-2022 | CN | 117396235 A | 12-01-2024 |
| | | EP | 4346917 A1 | 10-04-2024 |
| | | JP | 2024520406 A | 24-05-2024 |
| | | US | 2025009468 A1 | 09-01-2025 |
| | | WO | 2022248691 A1 | 01-12-2022 |
| US 2003018373 A1 | 23-01-2003 | AU | 2002344752 A1 | 02-01-2003 |
| | | CA | 2447881 A1 | 27-12-2002 |
| | | CN | 1531449 A | 22-09-2004 |
| | | EP | 1395338 A2 | 10-03-2004 |
| | | EP | 2295112 A1 | 16-03-2011 |
| | | JP | 4790982 B2 | 12-10-2011 |
| | | JP | 5148560 B2 | 20-02-2013 |
| | | JP | 2005508664 A | 07-04-2005 |
| | | JP | 2009261957 A | 12-11-2009 |
| | | US | 2003017073 A1 | 23-01-2003 |
| | | US | 2003018373 A1 | 23-01-2003 |
| | | US | 2003031586 A1 | 13-02-2003 |
| | | US | 2004034398 A1 | 19-02-2004 |
| | | WO | 02102418 A2 | 27-12-2002 |
| | | WO | 02102419 A2 | 27-12-2002 |
| US 2022323787 A1 | 13-10-2022 | KEINE | | |
| US 2015174426 A1 | 25-06-2015 | CN | 104582739 A | 29-04-2015 |
| | | EP | 2874667 A1 | 27-05-2015 |
| | | JP | 6258934 B2 | 10-01-2018 |
| | | JP | 2015524292 A | 24-08-2015 |
| | | US | 2015174426 A1 | 25-06-2015 |
| | | WO | 2014013385 A1 | 23-01-2014 |

Für nähere Einzelheiten zu diesem Anhang : siehe Amtsblatt des Europäischen Patentamts, Nr.12/82

EPO FORM P0461

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102009015088 A